# EUROPEAN PATENT APPLICATION

(11) **EP 4 733 269 A2**
(43) Date of publication of application: **29.04.2026**
(21) Application number: 26164263.1
(22) Date of filing: 24.11.2022
(51) Int. Cl.: C02F 1/00

(54) **PROCESS ANALYZING ARRANGEMENT**

(62) Divisional of application: 22209354.4
(71) Applicant: Hach Lange GmbH, 14163 Berlin (DE)
(72) Inventor: RUDDE, Heinz, 14163 Berlin (DE); HAHN, Markus, 14163 Berlin (DE); BRUBAKER, Silke, 14163 Berlin (DE); HEUWOLD, Maik, 14163 Berlin (DE); GOLITZ, Andreas, 14163 Berlin (DE); LEYSEN, Bas, 14163 Berlin (DE); DE HEIJ, Bas, 14163 Berlin (DE); KÜPPERS, Michael, 14163 Berlin (DE)
(74) Representative: terpatent PartGmbB

(57) **Abstract**

The invention is directed to a process analyzing arrangement (10) for continuously determining a water parameter of water (14) of a stationary water site (12), with an automatic analyzer unit (100) and a water sample feed pump (30) for pumping a water sample flow from the stationary water site (12) to the automatic analyzer unit (100),

The automatic analyzer unit (100) comprises:
a physical analyzer (66) with an analyzing chamber (60) where the water parameter is physically determined,
a separate grab sample container (110) of at least 20 ml for collecting a grab sample volume, and
a fluidic control arrangement (200) for selectively directing from the water sample flow an analyzer sample to the analyzing chamber (60) and/or a grab sample to the grab sample container (110),
wherein the fluidic control arrangement (200) comprises an electronic control unit (90) with a grab sampling control (92) and a grab sampling criterion means (94,95,96) for initiating a grab sample action controlled by the grab sampling control (92).

Since the automatic analyzer unit can provide a grab sample, no manual grab sampling is necessary anymore.

## Description

The invention refers to a process analyzing arrangement for continuously determining a water parameter of water of a stationary water site.

A process analyzing arrangement typically comprises a sample probe in a water basin with, for example, drinking water or sewage water and an automatic analyzer unit remote from the sample probe and the water basin. A water sample feed pump is provided for continuously pumping a water sample flow from the sample probe to the automatic analyzer unit where one or more parameter of the water is quasi-continuously physically determined in a physical analyzer for example in a photometer, fluorometer, electrochemical measurement cell, turbidimeter etc. Typical water parameters being controlled in a process analyzing arrangement are ammonium, phosphate, nitrate, COD etc.

A typical process analyzer is disclosed in US 2022 0136961 A1, US 6021664 A, US 2016 0340204 A1 and WO 2018 232 505 A1.

The results of the process analyzing arrangement are verified from time to time by manually taking a water grab sample from the stationary water site and by manually determining the relevant water parameter in a separate laboratory analyzer.

It is an object of the invention to simplify the verification of the results of a process analyzing arrangement.

This object is solved with a process analyzing arrangement with the features of claim 1.

The process analyzing arrangement according to the invention, for continuously determining a water parameter of water of a stationary water site, is provided with an automatic analyzer unit and a water sample feed pump for pumping a water sample flow from the stationary water site to the automatic analyzer unit. A feed pump is necessary because the automatic analyzer unit normally is located remote from the stationary water site. The water sample feed pump generally can be any type of pump, either a volumetric pump or a flow pump. Typically, the water sample feed pump is a peristaltic pump.

The automatic analyzer unit comprises a physical analyzer with an analyzing chamber where the water parameter is physically determined. The physical analyzer can be a photometer, a fluorometer, an electrochemical measurement cell, a turbidimeter or another type of a physical analyzer. A typical water parameter which is determined by the physical analyzer is ammonium, phosphate, nitrate, COD, the turbidity, or others.

The automatic analyzer unit is provided with a separate grab sample container of at least 20 ml for collecting a grab sample volume. The grab sample container is provided separately from the physical analyzer and allows to receive and to save a grab sample of the water which is pumped by the feed pump to the automatic analyzer unit. Preferably, the grab sample container has a volume of 50 to 200 ml.

The automatic analyzer unit is provided with a fluidic control arrangement for selectively directing an analyzer sample from the water sample flow coming from the stationary water site to the analyzing chamber of the physical analyzer and/or for selectively directing a grab sample to the grab sample container. The fluidic control arrangement comprises several valves which allow to fluidically direct the water sample flow alternatively or synchronously to the analyzing chamber and the grab sample container.

The fluidic control arrangement also comprises an electronic control unit with a grab sampling control and a grab sampling criterion means for initiating a grab sample action controlled by the grab sampling control. The grab sampling criterion means causes a grab sampling action.

The grab sampling criterion means preferably can be a manual trigger means which allows to manually initiate and trigger to pump water from the water sample flow into the grab sample container.

Preferably, the grab sampling criterion means can be an electronic grab time memory which defines a point or points in time for causing to pump water from the water sample flow into the grab sample container. The electronic control unit initiates a grab sampling action, for example, every 24 hours at the same time every day and with a sufficiently high grab sample volume to allow to manually supervise once a day the correct operation of the process analyzing arrangement. Alternatively, the electronic control unit can initiate a grab sampling action every single hour with a relatively small grab sample volume to finally provide a qualified grab sample after, for example 24 hours, representing a 24h-average of the water parameter in question.

Preferably, the grab sampling criterion means is a water parameter limit value memory which memorizes a limit value of the parameter in question. If the automatic analyzer unit detects an extreme value of the water parameter, a grab sampling action is initiated to allow to later manually verify if and to what extent the water parameter in question exceeded the limit value.

Alternatively or additionally, a second criterion can be monitored and can cause a grab sampling. A second criterion can be, for example, a pressure, a flow, a temperature or a pH-value.

According to a preferred embodiment, the grab sample container is provided at the outside of an analyzer unit housing of the automatic analyzer unit. More preferably, the grab sample container is provided simply removably at the automatic analyzer unit and is located outside of the analyzer unit housing. The sample container can manually be taken off and removed from the automatic analyzer unit without using any tool. However, the access to the grab sample container can be secured by a key lock so that the grab sample container can only be removed after being made accessible or after being released by using the corresponding key.

Preferably, the grab sample container has an excess top opening, and the automatic analyzer unit is provided with a grab sample supply line which leads into the access top opening. When the grab sample container is removed from the automatic analyzer unit, the grab sample supply line remains at the automatic analyzer so that only the grab sample container is removed from the automatic analyzer unit. The grab sample container can simply be removed from the automatic analyzer unit and can be brought to a separate laboratory analyzing device for providing a determination of the water parameter in question or of another water parameter.

As an alternative or in addition to a manual determination of the water parameter in a separate laboratory analyzer device, the physical analyzer of the automatic analyzer unit itself can provide the determination of the water parameter. This can be indicated, for example, if the grab sample container contains a qualified grab sample being an accumulation of many small grab sample volumes, or, if the liquid in the grab sample container is a standard liquid with a known value of the parameter.

Preferably, the automatic analyzer unit comprises a separate volumetric dosage pump for pumping a dosed water sample volume to the physical analyzer as well as to the grab sample container. The dosage pump typically has a displacement volume of 1 to 10 ml and preferably is a piston pump. A piston pump allows to very precisely pump small volumes from the water sample flow to the physical analyzer which allows an economical consumption of a reagent and to pump small volumes to the grab sample container which allows to provide a qualified grab sample.

Generally, the water sample feed pump can be located remote from the automatic analyzer unit. However, preferably, the water sample feed pump is integrated into the automatic analyzer unit so that all pumps of the process analyzing arrangement are part of the automatic analyzer unit.

Preferably, a sample probe is located at the stationary water site being located remote from the automatic analyzer unit. The sample probe can be provided with efficient filter means for mechanically clarifying the water sample flow flowing from the sample probe to the automatic analyzer unit.

Preferably, the automatic analyzer unit is provided with a reagent tank comprising a liquid reagent. The liquid reagent is added to the water sample for causing a reaction which allows to determine the water parameter in question in the analyzing chamber.

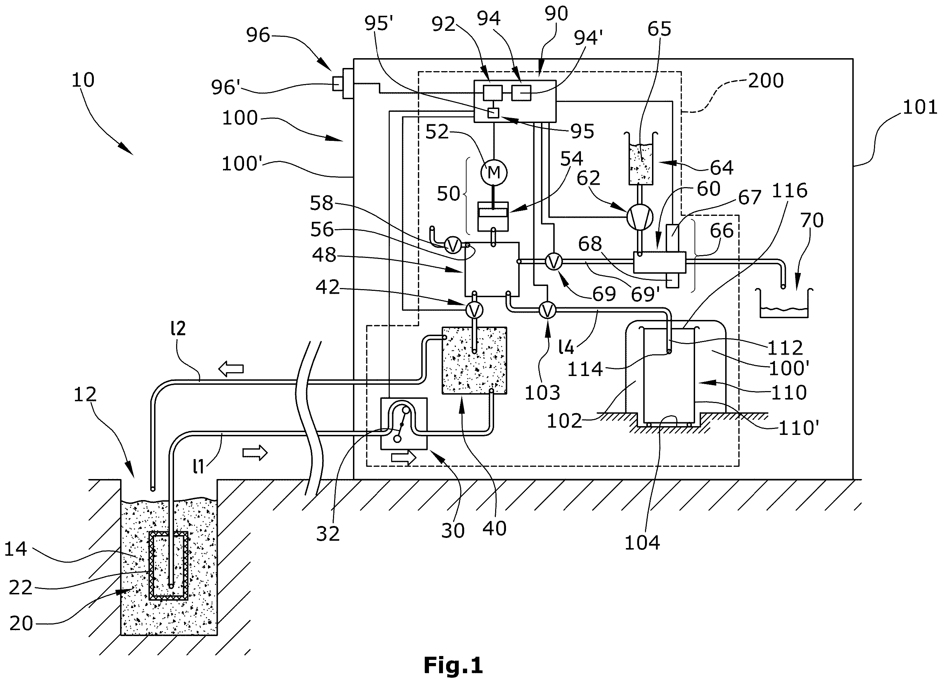

The invention is explained with reference to the enclosed drawing. The figure shows schematically a process analyzing arrangement with a stationary water site and an automatic analyzer unit located remote from the water site.

The figure schematically shows a process analyzing arrangement 10 with a stationary water site 12, which is, in the present embodiment, a water treatment basin of a water treatment plant, and shows a separate automatic analyzer unit 100 being located remote from the water site 12. The water basin comprises water 14 of which a water parameter is quasi-continuously controlled by the automatic analyzer unit 100. A typical water parameter is ammonium, phosphate, nitrate, or COD.

The process analyzing arrangement 10 comprises a sample probe 20 which is immersed into the water 14 at the water site 12 and which is provided with a filter element 22. The sample probe 20 is fluidically connected to the automatic analyzer unit 100 by a fluidic feed line l1.

The automatic analyzer unit 100 comprises within an analyzer unit housing 101 a comprehensive fluidic control arrangement 200 for controlling the sampling process within the automatic analyzer unit 100. One part of the analyzer unit housing 101 is a front panel 100' with a manual trigger means 96' and a grab sample container 110 both of which are easily accessible from the outside for a user person. The grab sample container 110 has an excess top opening 116 at the top, and is received in a container receptacle 102 including a container recess 104 for safely storing the container at the automatic analyzer unit 100. The grab sample container 110 can manually be taken off from the container receptacle 102 without any without using any tool. However, the grab sample container 110 can be secured at the container receptacle 102 with a key lock (not shown).

The water 14 is continuously pumped from the water site 12 via the fluidic feed line l1 by an electric water sample feed pump 30 into an intermediate chamber 40 from where the water flows continuously back to the water site 12 via a fluidic backflow line l2. The water sample feed pump 30 is a peristaltic hose pump with a pump rotor 32. The water sample feed pump 30 and the intermediate chamber 40 are part of the automatic analyzer unit 100 and of the fluidic control arrangement 200.

The intermediate chamber 40 is fluidically connected to a dosage chamber 48 via a fluidic line comprising an inlet valve 42. A separate volumetric dosage pump 50 is provided at the vertical top of the dosage chamber 40. The dosage pump 50 is a piston pump comprising a pump cylinder 54 and an electric piston actuator 50 for actuating a pump piston which is provided slidable within the pump cylinder 54. The displacement of the dosage pump 50 is 3,0 ml.

A venting line 56 with a venting valve 58, an analyzer line 69' with an analyzer line valve 69 and a grab line l4 with a grab line valve 103 are fluidically connected to the dosage chamber 48. The grab line l4 leads into a vertical grab sample supply line 112 with a downwardly orientated outlet opening 114, which grab sample supply line 112 is dived through the opening 114 into the interior of the grab sample container 110.

The analyzer line 69' fluidically connects the dosage chamber 48 with a physical analyzer 66, which physical analyzer 66 is, in the present embodiment, a photometer arrangement comprising a fluidic measurement cell 60, a photometric emitter 67 and a photometric receiver 68. The automatic analyzer unit 100 also comprises a reagent tank 64 comprising a suitable liquid reagent 65. The reagent tank 64 is fluidically connected to the fluidic measurement cell 60 via a volumetric reagent pump 62. A liquid waste container 70 is provided fluidically downstream of the fluidic measurement cell 60. The reagent 65 causes a colour reaction with the relevant water parameter, so that the concentration of the relevant water parameter can be determined with the physical analyzer 66 by measuring the transmission or the absorption at one or more specific wavelengths.

The automatic analyzer unit 100 comprises an electronic control unit 90 for controlling the valves 42, 69, 58, 103, the pumps 30, 50, 62 and the physical analyzer 66. The electronic control unit 90 comprises a grab sampling control and also comprises three different grab sampling criterion means 94, 95, 96 for initiating a grab sample action.

The first grab sampling criterion means 96 simply is a manual trigger means 96' at the front panel 100'. If the manual trigger means 96' is touched manually, a grab sampling action is immediately initiated and started by the grab sampling control 92.

The second grab sampling criterion means 95 is a water parameter limit value memory 95' memorizing at least one parameter limit value for the relevant water parameter. If the determination of the relevant valid parameter provided by the physical analyzer 66 exceeds the parameter limit value, a grab sampling action is immediately initiated and started by the grab sampling control 92. The memorized parameter limit value can be used to ensure that, in case of an extreme parameter value which is outside of the measurement range of the physical analyzer 66, the true value of the parameter value can be determined more precisely in a second (manual) determination of the parameter in question of the water sample in the grab sample container 110.

The third grab sampling criterion means 94 is an electronic grab time memory 94' which defines one or more fixed grab points in time and the volume of a single grab sample. The third grab sampling criterion means 94 can be used, for example, to provide a 24h qualified grab sample with a grab sampling action every 60 minutes.

During the measurement operation, the water 14 is continuously pumped by the water sample feed pump 30 from the stationary water site 12 to the intermediate chamber 40 and back through the backflow line l2 to the water site 12. The dosage pump 50 sucks water from the intermediate chamber 40 into the dosage chamber 48, and then pushes a relatively small volume of, for example, 10 ml into the analyzing chamber 60 where the reagent 65 is added to the liquid in the analyzing chamber 60. After having waited a reaction time of, for example, 10 minutes, the physical analyzer 66 determines the concentration of the parameter in question. The dosage chamber 84 is emptied, and a new measurement cycle is started.

When a grab sample action is initiated by one of the grab sampling criterion means 94, 95, 96, the dosage pump 50 pushes a suitable grab sample volume Vg from the dosage chamber 48 into the grab sample container 110.

The present embodiment schematically shows one single grab sample container 110. However, the automatic analyzer unit 100 can be provided with two or three separate grab sample containers, each of them for one of the three described grab sampling types.

## Claims

1. A process analyzing arrangement (10) for continuously determining a water parameter of water (14) of a stationary water site (12), with an automatic analyzer unit (100) and a water sample feed pump (30) for pumping a water sample flow from the stationary water site (12) to the automatic analyzer unit (100),
the automatic analyzer unit (100) comprising:
a physical analyzer (66) with an analyzing chamber (60) where the water parameter is physically determined,
a separate grab sample container (110) of at least 20 ml for collecting a grab sample volume, and
a fluidic control arrangement (200) for selectively directing from the water sample flow an analyzer sample to the analyzing chamber (60) and/or a grab sample to the grab sample container (110),
wherein the fluidic control arrangement (200) comprises an electronic control unit (90) with a grab sampling control (92) and a grab sampling criterion means (94,95,96) for initiating a grab sample action controlled by the grab sampling control (92).

2. The process analyzing arrangement (10) of claim 1, wherein the grab sampling criterion means (96) is a manual trigger means (96').

3. The process analyzing arrangement (10) of one of the preceding claims, wherein the grab sampling criterion means (94) is an electronic grab time memory (94') which defines the grab point in time (t) and the volume (Vg) of a grab sample.

4. The process analyzing arrangement (10) of one of the preceding claims, wherein the grab sampling criterion means (95) is a water parameter limit value memory (95').

5. The process analyzing arrangement (10) of one of the preceding claims, wherein the grab sample container (110) is provided at the outside of an analyzer unit housing (100').

6. The process analyzing arrangement (10) of one of the preceding claims, wherein the grab sample container (110) is provided removably at the automatic analyzer unit (100).

7. The process analyzing arrangement (10) of one of the preceding claims, wherein the grab sample container (110) has an access top opening (116), and a grab sample supply line (112) leads into the access top opening (116).

8. The process analyzing arrangement (10) of one of the preceding claims, wherein the automatic analyzer unit (100) comprises a separate volumetric dosage pump (50) for pumping a dosed water sample volume (Vd) to the analyzer (66).

9. The process analyzing arrangement (10) of one of the preceding claims, wherein the volumetric dosage pump (50) is a piston pump with a displacement volume of less than 10.1 ml.

10. The process analyzing arrangement (10) of one of the preceding claims, wherein the water sample feed pump (30) is integrated into the automatic analyzer unit (100).

11. The process analyzing arrangement (10) of one of the preceding claims, comprising a sample probe (20) being located at the stationary water site (12) remote from the automatic analyzer unit (100).

12. The process analyzing arrangement (10) of one of the preceding claims, wherein a grab container suction line (114) is provided to allow to pump a water sample from the grab sample container (110) to the physical analyzer (66).

13. The process analyzing arrangement (10) of one of the preceding claims, wherein the automatic analyzer unit (100) is provided with a reagent tank (64) comprising a liquid reagent (65), the fluidic control arrangement selectively directing the liquid reagent (65) from the reagent tank (64) to the analyzer sample.
